# EUROPEAN PATENT SPECIFICATION

(11) **EP 1 356 804 B1**
(45) Date of publication and mention of the grant of the patent: **01.07.2009**
(21) Application number: 02716387.2
(22) Date of filing: 25.01.2002
(51) Int. Cl.: A61K 8/22, A61K 8/29, A61Q 11/00, A61K 8/24, A61K 8/55

(54) **TOOTH BLEACHING AGENTS AND METHOD OF BLEACHING TEETH**
ZAHNBLEICHMITTEL UND VERFAHREN ZUM BLEICHEN VON ZÄHNEN
AGENTS ET PROCEDE DE BLANCHIMENT DES DENTS

(30) Priority: 29.01.2001 JP 2001020780; 23.03.2001 JP 2001084846
(43) Date of publication of application: 29.10.2003
(73) Proprietor: MITSUBISHI GAS CHEMICAL COMPANY, INC., Tokyo 100-0005 (JP); Japan Industrial Technology Association, Tokyo 105-0001 (JP); Nonami, Toru, Nagoya-shi Aichi-ken 464-0016 (JP)
(72) Inventor: NONAMI, Toru, Nagoya-shi, Aichi 464-0016 (JP); KAKUTA, Minoru, Mitsubishi Gas Chemical Co.Inc., Tsukuba-shi, Ibaraki 300-4247 (JP); SOMEYA, Masao, Mitsubishi Gas Chemical Co.Inc., Tsukuba-shi, Ibaraki 300-4247 (JP); OGASAWARA, Masumi, Mitsubishi Gas Chemical Co.Inc., Tsukuba-shi, Ibaraki 300-4247 (JP); ISHIBASHI, Takuro, Kodaira-shi, Tokyo (JP); ISHIBASHI, Kouzo, Kodaira-shi, Tokyo, (JP)
(74) Representative: Gille Hrabal Struck Neidlein Prop Roos
(86) International application number: PCT/JP2002/000556
(87) International publication number: WO 2002/060401

(56) References cited:
- EP-A- 1 048 291
- EP-A- 1 192 933
- EP-A1- 1 048 291
- WO-A1-01/01943
- WO-A1-95/17158
- WO-A1-99/02126
- US-A- 5 902 568

## Description

### TECHNICAL FIELD

The present invention relates to a bleaching agent for bleaching and removing colored sediment (pigmentation, discolored) on a tooth, and a bleaching method. More particularly, the present invention relates to a bleaching agent and a bleaching method **characterized in that** a bleaching agent comprising certain compositions having photocatalytic activity is applied onto a surface of a discolored tooth and then the tooth is bleached through photocatalytic action initiated by irradiation of the applied area with light.

### BACKGROUND ART

Recent years, there have been increasing demand for esthetic improvements of a tooth such as improvements of contours, alignment and integrity of a tooth in dental therapy. In particular, there have been more cases of young women desiring such dental therapy so as to whiten a tooth as an important element of beauty. Generally, the causes of dental discoloration, pigmentation or stain fall into the following categories: (1) extrinsic causes such as sedimentation of colored materials (tobacco, tea stain, etc.), pigment generating bacteria, discoloration of filling materials (primarily composite resins) and metal salts (primarily amalgam, silver nitrite, and ammonia silver); and (2) intrinsic causes such as aging, chemicals or medicine (ex. fluorine, tetracycline and the like), dysmmetabolism and hereditary, and dental injuries.

Several methods have been proposed as methods for esthetic improvement of a discolored tooth, among which bleaching may be considered as a highly effective method for the preservation of dentine although there may be some cases of color reversion and so on. The bleaching method is basically a method for decolorizing colored materials through a chemical reaction. In the past, there have been various reports of bleaching agents comprising a variety of chemicals based on a vital bleaching method and a non-vital bleaching method as well as bleaching methods using such agents.

The following are typical examples:
(1) Bleaching method using a 30% H₂O₂ solution as a chemical agent in combination with light and heat in parallel.
   This is a method in which a strip of gauze soaked in a 30% H₂O₂ solution is placed on the front surface of a tooth and is irradiated for 30 minutes from the left and right by using a pair of 500-W photographic illumination lamps. In this method, the lamps are set as close to the gauze as possible and the H₂O₂ solution should be replenished about every 5 minutes so as to prevent it from drying.
(2) Bleaching method using a 30% H₂O₂ solution as a chemical agent in combination with a high frequency electric current in parallel.
   This is a method in which a strip of gauze soaked in a 30% H₂O₂ solution placed on the front surface of a tooth and high frequency electric current is fed for 1 second with a spoon-shaped tip of a high frequency electric scalpel and is discontinued for 8 second. The operation should be repeated 6 to 8 times, and the H₂O₂ solution should be replenished when the gauze is dried during the operation.
(3) Bleaching method using a paste comprising Aerosil (fine silica powder) mixed with a 35% H₂O₂ solution.
   This is a method in which the paste is applied onto the surface of an etched tooth, 15 minutes later the tooth is rinsed with water and then polished. The method gives a highly bleaching result without using light or heat as Aerosil serves as a moisturizing material to prevent the bleaching agent from drying out and also enhances the bleaching effects. However, the 35% H₂O₂ solution should be handled with care due to its highly corrosive property.
(4) Bleaching method using a paste (Shofu Hi-Lite : tradename), obtained by kneading a 35% H₂O₂ solution and a powder comprising potassium sulfate, manganese sulfate, silicon dioxide or the like, as a chemical agent.
   This is a method in which the paste is placed on the front surface of a tooth, and is worked for 10 minutes or irradiated with light for 3 minutes by applying a visible light beam radiation device. An advantage of the method is that the paste shows a light green immediately after the kneading and then turns yellow by the light irradiation. Further, if the paste turns dark-brown immediately after the kneading, this indicates that the bleaching effects of the liquid have diminished. However there is the same disadvantage mentioned above due to the use of the 35% H₂O₂ solution.
(5) Bleaching method using a mixed solution composed of 1 ml of 30% HCl, 1 ml of 30% H₂O₂, and 0.2ml of diethyl ether as a chemical agent (Improved Machines bleaching method).
   In this method, the mixture is allowed to act for 5 minutes on a tooth surface, and then the tooth is polished for 15 minutes under a small pressure using a polishing disc. The treatment is repeated 3 times, then it is neutralized with 5.25% NaOCl and the tooth is thoroughly rinsed with water (Oral Surg., 26: 871-878(1968), J. Am. Dent. Assoc., 87: 1329(1973)). In this method, since the paste may scatter into the patient's eyes, it is necessary to care for protecting them from it adequately.
(6) Bleaching method using a paste obtained from kneading a sodium perborate powder and a 30% H₂O₂ solution (Walking bleach method).
   In this method, so as to enlarge the dentinal tubule and enhance the bleaching effects, the inner walls of the pulp cavity are treated with phosphoric acid for 1 minute followed by rinsing with water and drying. Then the paste is introduced into the pulp cavity and temporarily sealed with cement. Although this method is currently, widely applied for clinical purposes to which the Public Health Insurance is applicable as a simple and highly effective method, it's disadvantage is the same as that mentioned above due to the use of the 30% H₂O₂ solution.

There are other many bleaching methods reported including the following:
A bleaching agent comprising a mixture of aqueous hydrogen peroxide and ortho-phosphoric acid, and the bleaching method (Japanese Patent Application Laid-Open No. H8-143436/1996).
A bleaching agent obtained from mixing silicic acid anhydride with aqueous hydrogen peroxide and the vital bleaching method characterized by applying the bleaching agent (Japanese Patent Application Laid-Open No. H5-320033/1993).
A dental bleaching compositions comprising a dental bleaching agent (hydrogen-urea peroxide, hydrogen peroxide-carbamide, carbamide peroxide, and the like) and a matrix (carboxymethylen and the like), and a bleaching method using the dental bleaching compositions (Japanese Patent Application Laid-Open No. H8-113520/1996).

On the other hand, bleaching methods and bleaching agents for dental bleaching require the following conditions:
(a) pronounced bleaching results,
(b) no toxicity of bleaching agents,
(c) easy operations,
(d) no degradation to dental physical properties after bleaching,
(e) efficacious for vital tooth bleaching as well as non-vital tooth bleaching,
(f) speedy bleaching results, and so on.

However, in conventional bleaching agents or methods, the primary bleaching agent is 30 wt. % to 35 wt. % aqueous hydrogen peroxide and its oxidative property is the base for bleaching a tooth. Therefore there are difficulties of simplicity and safety of operation. There are other bleaching methods with the use of 10 wt. % urea peroxide solution in lieu of 30 wt. % to 35 wt. % aqueous hydrogen peroxide; however, this method has also been litigated over the problems in terms of medical virtues and safety, and has not obtained the official approval in Japan.

Taking the aforementioned status of the bleaching methods into consideration, the inventors of the present invention have found that, as new bleaching agents and bleaching methods showing excellency in safe and simplicity as well as showing remarkable efficacious to a vital tooth as well as a non-vital tooth within a short period without the use of highly toxic 30 wt. % to 35 wt. % aqueous hydrogen peroxide, a bleaching agent using titanium oxide having photocatalytic action and low concentration aqueous solution of hydrogen peroxide in parallel is effective (Japanese Patent Application Laid-Open No. H11-92351/1999).

However, the storage stability of the tooth bleaching agents is inadequate: 20 % of hydrogen peroxide component varnishes after a month storage under refrigeration at the temperature of 5 °C to 8 °C. Further, there happened to show inadequate bleaching result caused by difficulty of applying it onto a tooth surface due to the reduction of efficacy of the thickening agent. Therefore, a tooth bleaching agent having better storage stability have been desired.

### DISCLOSURE OF THE INVENTION

It is the objectives of the present invention to provide that a bleaching agent, which overcomes the problems aforementioned, being good in long-term storage stability, easy to apply it onto a discolored tooth on practical application and achieves adequate bleaching results without deteriorating original photocatalytic activity, further is excellent in safety too. The present inventors were dedicated to joining the research of the above tooth bleaching agents and then have found out that an inorganic thickening agent or an organic thickening agent with phosphoric acid and condensed phosphate contained in a bleaching agent would improve long-term storage stability, conveniences for applying it onto a discolored tooth on practical application and also obtain satisfactory bleaching results without deteriorating original photocatalytic activity, further it would be excellent in safety too. The present invention was achieved based on the foregoing findings.

Namely, the present invention provides a tooth bleaching agent comprising (a) titanium dioxide initiating photocatalytic action with light irradiation, (b) a chemical compound generating hydrogen peroxide in aqueous solution, (c) an inorganic thickening agent or an organic thickening agent, (d) phosphoric acid and (d) condensed phosphate. Further, the present invention provides a tooth bleaching method characterized by bleaching a tooth by applying the bleaching agent onto the surface of a tooth followed by irradiating the applied surface with light.

### PREFERRED EMBODIMENT OF THE INVENTION

The tooth bleaching agent of the present invention comprises (a) titanium dioxide initiating photocatalytic action with light irradiation, (b) a chemical compound generating hydrogen peroxide in aqueous solution, (c) (c1) an inorganic thickening agent or (c2) an organic thickening agent, (d) phosphoric acid and (d) condensed phosphate.

In the case where (c) is (c1) an inorganic thickening agent, it is preferable that the tooth bleaching agent comprises two types of liquid, [liquid-1] and [liquid-2], wherein [liquid-1] is a mixed solution of (a) titanium oxide initiating photocatalytic action with light irradiation and (c1) an aqueous solution of an inorganic thickening agent, and wherein [liquid- 2] is a mixed aqueous solution of (b) a chemical compound generating hydrogen peroxide in aqueous solution with (d) phosphoric acid and (d) condensed phosphate. Namely, mixing [liquid-1] with [liquid-2], and applying it onto a discolored tooth surface followed by irradiating thereon with light to bleach the discolored tooth, both a good long-term storage stability at room temperature and an excellent bleaching result to a discolored tooth can be obtained.

In addition, in the case where (c) is (c2) an organic thickening agent, a mixture of (a) component to (d) component is applied onto the surface of a discolored tooth and then the discolored tooth is bleached through irradiation of the tooth with light. Then, excellent bleaching results with outstanding long-term stability at room temperature are realized.

Any types of (a) titanium dioxide, without regard to its form or property, are possible to be employed for the present invention only if it initiates the photocatalytic action. The preferable examples of the titanium dioxide include anatase-type, the rutile-type and the brookite-type. The rutile-type is particularly preferable. Further, the foregoing titanium oxide of which the affinity to a tooth surface being improved by coating the surface of itself with calcium phosphate may be employed. Furthermore, the titanium dioxide of which photocatalytic activity being improved by depositing platinum on it or the titanium dioxide treated with plasma and the like thereby initiating the photocatalytic action by visible light may be employed.

Titanium dioxides of a powder-type or a sol-type obtained from dispersing it into a medium such as water can be employed too. The titanium dioxide having the particle diameter of from 1 nm to 500 nm is suitable for the use, further the particle diameter of from 5 nm to 200 nm is more preferable to it. A small amount of the titanium dioxide content shows adequate results. Namely, the preferred content of titanium dioxide in the bleaching agent is from 0.001 % by weight to 10 % by weight, more preferably from 0.01 % by weight to 1 % by weight, and the content of from 0.01 % by weight to 0.1 % by weight is further more preferable. Depending on the intensity of tooth discoloration, a very small amount of the titanium dioxide content takes possibly longer time so as to obtain reasonable results. On the other hand, too high content of it inversely causes lowering the bleaching effects due to an inferior light permeability of the titanium dioxide.

As a chemical compound generating hydrogen peroxide in aqueous solution being used for the present invention, any chemical compound is worked only if it generates hydrogen peroxide by making its aqueous solution. Although hydrogen peroxide, perborate, percarbonate, perphosphate, persulfate, calcium peroxide, magnesium peroxide, urea peroxide, and the like are nominated, hydrogen peroxide is preferable.

In the tooth bleaching agents of the present invention, even a very lower concentration of a chemical compound generating hydrogen peroxide by comparison with conventional bleaching agents is able to show remarkable bleaching results. Namely, the content of the chemical compounds generating hydrogen peroxide in aqueous solution is preferably 35% by weight or less, more preferably 1% to 10% by weight. Even if the content is higher than the range, there would be not much difference in the bleaching results but it has disadvantages in view of safety.

As (c1) inorganic thickening agents being used for the present invention, inorganic clay minerals, more preferably layer-structure inorganic clay minerals are used.

Generally, inorganic clay minerals are roughly classified into a fibrous structure type (ex. sepiolite, attapulgite, etc.), a non-crystal structure type (ex. allophane, etc.), mixed-layers structure type (ex. kaolinte, montmorillonite, etc.) and the above layer-structure type. Inorganic clay minerals of the layer-structure type take water molecules into a unit space between the layers, and then swell. By utilizing this property, the hydrogen peroxide existing in the bleaching system is to be held as adhered onto the surface of a discolored tooth. In the present invention, the inorganic clay minerals of the layer-structure type, of which property is to swell in the presence of water, are preferable to use.

Even if clay minerals are the inorganic clay minerals of a fibrous structure type or a non-crystal structure type, swelling thereof is realized by adding water to them followed by mixing them with a high-speed mixer, however, the layer-structure clay minerals have a merit because of no such machine required.

The following are examples of inorganic clay minerals, but not limited to: dickite, nacrite, kaolinite, anorthite, halloysite, metahalloysite, chrysotile, lizardite, serpentine, antigorite, beidellite, montmorillonite, sauconite, stevensite, nontronite, saponite, hectorite, vermiculite, smecnite, sepiolite, nacrite, illite, sericite, glauconite-montmorillonite, roselite-montmorillonite, chlorite-vermiculite, illite-montmorillonite, halloysite-montmorillonite, kaolinite-montmorillonite and the like.

Among the above inorganic clay minerals, montmorillonite, sauconite, smectite, stevensite, beidellite, nontronite, saponite, hectorite, vermiculite, nacrite, sepiolite and the like are nominated as particularly preferable clay minerals of the layer-structure type for the present invention. Not only natural products but also synthesized products thereof are to be used, and also the mixtures of the two or more thereof are possible to use. In addition, the blending amount of these inorganic clay minerals in the mixture of (a) component to (d₁) and (d₂) component is preferably 0.1 by weight to 10 % by weight, more preferably 0.5 by weight to 5 % by weight.

Water-soluble polymer is employed as (c2) organic thickening agents in the present invention, and a thickening agent for food additives is preferably employed from the viewpoint of safety. Such water-soluble polymer achieves to maintain the contact of hydrogen peroxide existing in the system with a discolored tooth by utilizing the property of forming intermolecular or intra-molecular cross-linking structure and of thickening by the swell after absorbing water. It is preferable to use such water-soluble polymer being swelled in the presence of water in the present invention.

Examples of the water-soluble polymer include starch, galactomannan, cellulose nitrate, methyl cellulose, hydoxymethylcellulose, pectinic acid, alginic acid, agar, carageenin, proteoglycan, glycoprotein, gelatine, actin, tubulin, hemoglobin S, insulin, fibrin, egg albumin, serum albumin, myosin, collagen, polypeptides, casein, polyvinyl alcohol, polyethylene glycol, polyethylene oxide, polyacrylamide and the like.

Among the above water-soluble polymers, particularly preferable examples of a thickening agent for food additives in the present invention include sodium alginate, propylene glycol alginate, Sodium carboxymethylcellulose, Sodium carboxymethylstarch, Sodium starch phosphate, methyl cellulose, sodium polyacrylate and the like. Further preferably, sodium polyacrylate, methylcellulose and Sodium carboxymethylcellulose is used due to excellency in long-term storage stability thereof. Not only natural products but also synthesized products thereof are to be used, and also the mixtures of the two or more thereof are possible to be employed. In addition, the blending amount of these thickening agents in the mixture of the (a) component to (d₁) and (d₂) components is preferably 0.01 by weight to 10 % by weight, more preferably 0.1 by weight to 5 % by weight.

The (d₁) phosphoric acid and (d₂) condensed phosphate to be used for the present invention are used as a stabilization agent and a bleaching accelerator. Orthophosphoric acid is preferable as phosphoric acid. Preferable examples of the condensed phosphate include potassium salt, sodium salt, and the like of pyrophosphoric acid, tripolyphosphoric acid and so forth. In particular, tetra-sodium pyrophosphate is preferable.

The blending amount of the phosphoric acids in the mixture of the (a) component to the (d₁) and (d₂) components is 0.1 to 10 % by weight, preferably 0.2 to 2 % by weight. If the amount were less than the range, the bleaching result would be limited and if it were more than the range, the acidity of the bleaching composition would become stronger so that it happens to arise problems such as adverse effect to a tooth surface. Further, the blending amount of the condensed phosphate in the mixture of the (a) component to the (d₁) and (d₂) components is 0.1 by weight to 10 % by weight, preferably 0.5 by weight to 5 % by weight. If the amount were less than the range, the bleaching result would be limited and if it were more than the range, the liquid property would become alkaline so that the stability of hydrogen peroxide would be turned down.

The tooth bleaching agent of the present invention enables to keep long-term storage stability thereof, further to maintain its viscosity so as not to sag from a tooth of patient and as well as adequate amount of the bleaching component on the tooth. The viscosity of the bleaching agent is 0.001 Pa·S to 100 Pa·S, preferably 0.002 Pa·S to 50 Pa·S. When the viscosity is kept within the range above, the bleaching agent does not sag even in the case of applying it onto a tooth surface with the angle of 45 degrees to the horizontal. Although it is possible to storage the tooth bleaching agent of the present invention for long-term at room temperature, it is preferable to storage them at the temperature of from 1 °C to 10 °C in the dark place. Further, in case of the tooth bleaching agent of the two liquid component type comprising [liquid-1] and [liquid-2], it is preferable to storage them under a light-shielded condition.

As a method of applying the tooth bleaching agent of the present invention onto a tooth surface, it is preferable to apply them directly to the tooth surface. Further the bleaching of a discolored tooth is proceeded as follows: the bleaching agent above is applied to the tooth surface and then the applied area is irradiated with light at least once, preferably repeating more than once. The light to be used should contain a suitable wavelength being absorbed by titanium dioxide, then initiating photocatalytic action and also preferably it rarely has adverse affect to human body. Such a wavelength is explained with regard to the light having wavelength of 300 nm or longer, preferably 380nm to 500nm. The wavelength more than 500 nm is not preferable as it elevates the temperature of the tooth on a large scale. The examples of light source to be used for the light of the present invention are as the following: an incandescent lamp, a fluorescent lamp, a halogen light bulb, a black light, a metalhalide lamp, a xenon lamp, a mercury lamp, a UV lamp, a LED (Light Emitting Diode) lamp, a semiconductor laser, and the like. The light of these light sources from which unnecessary wavelengths are cut out by using a proper filter is guided and irradiated to a tooth surface applied by the bleaching agent. These applications and irradiation are repeated optionally corresponding to the intensity of a tooth discoloration. In case of applying operation such as applying the bleaching agent to a tooth surface, it would be done that the fresh agent is applied every about 15 to 20 minutes and the interval and frequency are properly decided corresponding to the condition of the tooth. The tooth bleaching agent of the present invention is effective to bleach a vital tooth as well as a non-vital tooth and show remarkable effects so as to bleach it safely and simply.

The main action of the tooth bleaching agents of the present invention is the bleaching action based on synergistic effect of the titanium dioxide photocatalyst, low concentration of hydrogen peroxide and the thickening agents. That is, irradiating titanium oxide with the light generates electrons and positive holes, and then these react with hydrogen peroxide leading to generation of active oxygen. The active oxygen shows significantly strong oxidative force; therefore, it is able to bring out the bleaching results through oxidative decomposition of the discolored sediment on a tooth surface. In addition, by exercise of a proper organic thickening agent, the bleaching agent applied to a tooth surface is kept without sagging and enough amounts for bleaching the discolored tooth can be supplied, accordingly the handling as well as the safety is much improved.

The present invention is explained in details by the following examples, but it is not restricted thereby.

### Example 1

[Liquid-2] which was prepared by adding purified water to a mixture of 9.94g of hydrogen peroxide with 35% by weight, 0.60g of phosphoric acid, and 2.00g of sodium pyrophosphate decahydrate so as to be 60g aqueous solution in total, was kept in storage for 4 months at the temperature of 25 °C, thereafter the decomposition rate of hydrogen peroxide was 0.2%.

### Comparative Example 1

The aqueous solution, which was prepared by adding purified water to a mixture of 9.94g of hydrogen peroxide with 35% by weight, 1.5g of an inorganic thickening agent (Nippon Silica Industrial Co., Ltd.: Laponite XLG), and 0.06g of the rutile-type titanium oxide (TYACA MT-150A) so as to be 100g aqueous solution in total, was kept in storage for 1 week at the temperature of 25 °C, thereafter the decomposition rate of hydrogen peroxide was 56%.

### Example 2

[Liquid-1], which was the mixture of 1.5g of an inorganic thickening agent (Nippon Silica Industrial Co., Ltd.: Laponite XLG), 0.06g of the rutile-type titanium oxide (TYACA MT-150A) and 38.44g of purified water, was mixed with the liquid-2 in Example 1, followed by keeping it in storage at the temperature of 25 °C. The decomposition rate of hydrogen peroxide was 10% after 1-week storage and 86% after 1-month storage.

### Example 3

As the model test for bleaching, a test liquid, which was prepared by solving 10ppm of methylene blue (blue pigment) into the bleaching agent comprising [liquid-2] in Example 1 and [liquid-1] in Example 2, was filled in a quartz cell of 1cm each side, followed by the irradiation with the light having wavelength of 380 nm to 500 nm for 5 minutes. The result thereof was 90 % of the methylene blue decomposition.

### Example 4

The same procedure of Example 3 was applied except the use of the rutile-type titanium oxide (Ishihara Techno TTO-55) in lieu of the rutile-type titanium oxide (TYACA MT-150A). The result thereof was 79 % of the methylene blue decomposition.

### Example 5

The same procedure of Example 3 was applied except the use of the anatase-type titanium oxide (Ishihara Techno ST-21) in lieu of the rutile-type titanium oxide (TYACA MT-150A). The result thereof was 22 % of the methylene blue decomposition.

### Example 6

The same procedure of Example 3 was applied except the use of the anatase-type titanium oxide (Shouwa Titanium F-6) in lieu of the rutile-type titanium oxide (TYACA MT-150A). The result thereof was 36 % of the methylene blue decomposition.

### Example 7

By using the bleaching agent prepared by mixing [liquid-2] in Example 1 and [liquid-1] in Example 2, bleaching of a discolored tooth (extracted tooth) was carried out as follows:
1) As preliminary arrangements, the plaque, the tartar, the tar and the like were removed by using a ultrasonic scaler.
2) The tooth surface was cleaned with rubber cup and the like, and then dried as a conventional practice.
3) A convenient moisture-proofing method was carried out.
4) The bleaching agent was applied onto the tooth surface followed by irradiation with light having the wavelength of 380 nm or longer.
5) An irradiation time was 5 minutes, and the application of the new bleaching agent above and the irradiation was carried out every turn: the operation was repeated 4 times.

The result thereof showed that the initial discoloration intensity of F3.5 was, thereafter, changed to the discolored intensity of F1.5, wherein the evaluation for the discoloration intensity of the used discolored tooth (extracted tooth) was classified as follows;
F1: entire crown uniformly colored light yellow, brown and gray with no striations.
F2: entire crown uniformly colored a deeper shade than F1, with no striations.
F3: deep gray and bluish gray with striations.
F4: entire crown discolored to extremely deep purple and grayish purple.

### Comparative Example 2

The result of bleaching the discolored tooth (extracted tooth) by using the same method as Example 7 except the use of the bleaching agent prepared in Comparative Example 1 showed that the initial discoloration intensity of F3.5 was, thereafter, changed to the discolored intensity of F2.0.

### Example 8

The tooth bleaching agent was prepared by adding purified water to a mixture of 16.57g with 35 wt. % hydrogen peroxide, 0.60g of phosphoric acid, 2.00g of sodium pyrophosphate decahydrate, 0.06g of the rutile-type titanium oxide (TYACA MT-150A) and 0.20g of sodium polyacrylate so as to be 100g aqueous solution in total. It was kept in storage for 110 days at the temperature of 25 °C, thereafter the decomposition rate of hydrogen peroxide was 8.5%.

### Example 9

The tooth bleaching agent was prepared by adding purified water to a mixture of 16.57g with 35 wt. % hydrogen peroxide, 0.60g of phosphoric acid, 2.00g of sodium pyrophosphate decahydrate, 0.06g of the rutile-type titanium oxide (TYACA MT-150A) and 0.20g of sodium polyacrylate so as to be 100g aqueous solution in total. It was kept in storage for 110 days at the temperature of 8 °C in the refrigerator, thereafter the decomposition rate of hydrogen peroxide was 2.7%.

### Example 10

The tooth bleaching agent was prepared by adding purified water to a mixture of 16.57g with 35 wt. % hydrogen peroxide, 0.60g of phosphoric acid, 2.00g of sodium pyrophosphate decahydrate, 0.06g of the rutile-type titanium oxide (TYACA MT-150A) and 1.00g of carboxymethylcellulose sodium so as to be 100g aqueous solution in total. It was kept in storage for 110 days at the temperature of 8 °C in the refrigerator, thereafter the decomposition rate of hydrogen peroxide was 0.6%.

### Example 11

As the model test for bleaching, a test liquid, which was prepared by solving 10ppm of methylene blue (blue pigment) into the bleaching agent prepared in Example 8, was filled in a quarts cell of 1cm each side followed by the irradiation with the light having the wavelength of 380 nm or longer for 10 minutes. The result thereof was 90 % of the methylene blue decomposition.

### Example 12

The result of bleaching the discolored tooth (extracted tooth) by using the same method as Example 7 except the use of the bleaching agent prepared in Example 8 showed that the initial discoloration intensity of F3.5 was changed, thereafter, to the discolored intensity of F1.5.

### INDUSTRIAL APPLICABILITY

The tooth bleaching agent of the present invention and the bleaching method thereby show marvellous results such as (1) excellent long-term storage and stability, (2) considerable improvement for workability on production and application thereof, (3) easy application onto a tooth surface, (4) high safety assured by good bleaching results with a low concentration of hydrogen peroxide, (5) remarkable bleaching effects and the like.

## Claims

1. A tooth bleaching agent comprising
(a) titanium dioxide initiating photocatalytic action by light irradiation,
(b) a chemical compound generating hydrogen peroxide in an aqueous solution,
(c) an inorganic thickening agent or an organic thickening agent,
(d1) phosphoric acid, and
(d2) condensed phosphate.

2. The tooth bleaching agent as defined in claim 1, comprising two solutions,
one of which is a solution-1 comprising an aqueous mixture of (a) titanium dioxide initiating photocatalytic action by light irradiation and (c1) an inorganic thickening agent and
the other one is a solution-2 comprising the mixed aqueous solution of (b) a chemical compound generating hydrogen peroxide in an aqueous solution, (d1) phosphoric acid and (d2) condensed phosphate.

3. The tooth bleaching agent as defined in claim 1, wherein it comprises
(a) titanium dioxide initiating photocatalytic action by light irradiation,
(b) a chemical compound generating hydrogen peroxide in an aqueous solution,
(c2) an organic thickening agent,
(d1) phosphoric acid and
(d2) condensed phosphate.

4. The tooth bleaching agent as defined in claim 1, wherein (a) titanium dioxide initiating photocatalytic action by light irradiation is a rutile type titanium dioxide.

5. The tooth bleaching agent as defined in claim 1, wherein (b) the chemical compound generating hydrogen peroxide in an aqueous solution comprises at least one kind of peroxide selected from the group consisting of hydrogen peroxide, perborate, percarbonate, persulfate, perphosphate, calcium peroxide, magnesium peroxide and urea peroxide.

6. The tooth bleaching agent as defined in claim 1, wherein (b) the chemical compound generating hydrogen peroxide in an aqueous solution is hydrogen peroxide.

7. The tooth bleaching agent as defined in claim 1, wherein the amount of (b) the chemical compound generating hydrogen peroxide in a aqueous solution contained in the agent is 35 % by weight or less.

8. The tooth bleaching agent as defined in claim 1, wherein the amount of (b) the chemical compound generating hydrogen peroxide in an aqueous solution contained in the agent is from 1 % by weight to 10 % by weight.

9. The tooth bleaching agent as defined in claim 1, wherein (c1) the inorganic thickening agent comprises at least one kind of inorganic clay mineral selected from the group consisting of saponite, montmorillonite, stevensite, hectorite, smectite, nacrite and sepiolite.

10. The tooth bleaching agent as defined in claim 1, wherein (c2) the organic thickening agent comprises at least one kind of material selected from the group consisting of sodium polyacrylate, methylcellulose and sodium carboxymethylcellulose.

11. A method of bleaching a tooth by applying the tooth bleaching agent as defined in any one of claims 1 to 10 onto the surface thereof and irradiating the applied surface with light.

12. The method as defined in claim 11, wherein the wavelength of the light for the irradiation is from 380 nm to 500 nm.

## Patentansprüche

1. Zahnbleichmittel, umfassend
(a) Titandioxid, das durch Bestrahlung mit Licht eine photokatalytische Wirkung auslöst,
(b) eine chemische Verbindung, die in wässriger Lösung Wasserstoffperoxid bildet,
(c) ein anorganisches Verdickungsmittel oder ein organisches Verdickungsmittel,
(d1) Phosphorsäure, und
(d2) kondensiertes Phosphat,

2. Zahnbleichmittel wie in Anspruch 1 definiert,
umfassend zwei Lösungen,
wobei eine von ihnen eine Lösung-1 ist, umfassend eine wässrige Mischung von (a) Titandioxid, das durch Bestrahlung mit Licht eine photokatalytische Wirkung auslöst, und (c1) ein anorganisches Verdickungsmittel, und wobei die andere eine Lösung-2 ist, umfassend das wässrige Lösungsgemisch von (b) einer chemischen Verbindung, die in einer wässrigen Lösung Wasserstoffperoxid bildet, (d1) Phosphorsäure und (d2) kondensiertes Phosphat,

3. Zahnbleichmittel wie in Anspruch 1 definiert, wobei es umfasst
(a) Titandioxid, das durch Bestrahlung mit Licht eine photokatalytische Winkung auslöst,
(b) eine chemische Verbindung, die in einer wässrigen Lösung Wasserstoffperoxid bildet,
(c2) ein organisches Verdickungsmittel,
(d1) Phosphorsäure und
(d2) kondensiertes Phosphat,

4. Zahnbleichmittel wie in Anspruch 1 definiert, wobei (a) Titandioxid, das durch Bestrahlung mit Licht eine photokatalytische Wirkung auslöst, ein Titandioxid vom Rutil-Typ ist.

5. Zahnbleichmittel wie in Anspruch 1 definiert, wobei (b) die chemische Verbindung, die in einer wässrigen Lösung Wasserstoffperoxid erzeugt, mindestens ein Peroxid umfasst, das ausgewählt wird aus der aus Wasserstoffperoxid, Perborat, Percarbonat, Persulfat, Perphosphat, Calciumperoxid, Magnesiumperoxid und Harnstoffperoxid bestehenden Gruppe.

6. Zahnbleichmittel wie in Anspruch 1 definiert, wobei (b) die chemische Verbindung, die in einer wässrigen Lösung Wasserstoffperoxid erzeugt, Wasserstoffperoxid ist.

7. Zahnbleichmittel wie in Anspruch 1 definiert, wobei die Menge von (b) der im Mittel enthaltenen chemischen Verbindung, die in einer wässrigen Lösung Wasserstoffperoxid erzeugt, 35 Gew.-% oder weniger beträgt.

8. Zahnbleichmittel wie in Anspruch 1 definiert, wobei die im Mittel enthaltene Menge von (b) der chemischen Verbindung, die in einer wässrigen Lösung Wasserstoffperoxid erzeugt, 1 Gew.-% bis 10 Gew.-% beträgt.

9. Zahnbleichmittel wie in Anspruch 1 definiert, wobei (c) das anorganische Verdickungsmittel mindestens eine Art eines anorganischen Tonmaterials umfasst, das ausgewählt wird aus der aus Saponit, Montmorillonit, Stevensit, Hectorit, Smectit, Nacrit und Sepiolit bestehenden Gruppe.

10. Zahnbleichmittel wie in Anspruch 1 definiert, wobei (c2) das organische Verdickungsmittel mindestens eine Art Material umfasst, das ausgewählt wird aus der aus Natriumpolyacrylat, Methylcellulose und Natriumcarboxymethylcellulose bestehenden Gruppe.

11. Verfahren zum Bleichen eines Zahns durch Anwendung des Zahnbleichmittels, wie in einem der Ansprüche 1 bis 10 definiert, auf dessen Oberfläche und Bestrahlen der behandelten Oberfläche mit Licht.

12. Verfahren wie in Anspruch 11 definiert, wobei die Wellenlänge des Lichts zur Bestrahlung von 380 nm bis 500 nm beträgt.

## Revendications

1. Agent de blanchiment des dents comprenant
(a) du dioxyde de titane initiant une action photocatalytique par irradiation lumineuse,
(b) un composé chimique générant du peroxyde d'hydrogène dans une solution aqueuse,
(c) un agent épaississant inorganique ou un agent épaississant organique,
(d1) de l'acide phosphorique, et
(d2) du phosphate condensé.

2. Agent de blanchiment des dents selon la revendication 1, comprenant deux solutions,
dont l'une est une solution-1 comprenant un mélange aqueux de (a) dioxyde de titane initiant une réaction photocatalytique par irradiation lumineuse et (c1) un agent épaississant inorganique et dont l'autre est une solution-2 comprenant la solution aqueuse mixte de (b) un composé chimique générant du peroxyde d'hydrogène dans une solution aqueuse, (d1) de l'acide phosphorique et (d2) du phosphate condensé.

3. Agent de blanchiment des dents selon la revendication 1, lequel comprend
(a) du dioxyde de titane initiant une action photocatalytique par irradiation lumineuse,
(b) un composé chimique générant du peroxyde d'hydrogène dans une solution aqueuse,
(c2) un agent épaississant organique,
(d1) de l'acide phosphorique et
(d2) du phosphate condensé.

4. Agent de blanchiment des dents selon la revendication 1, dans lequel (a) le dioxyde de titane initiant une action photocatalytique par irradiation lumineuse est un dioxyde de titane de type rutile.

5. Agent de blanchiment des dents selon la revendication 1, dans lequel (b) le composé chimique générant du peroxyde d'hydrogène dans une solution aqueuse comprend au moins un type de peroxyde choisi parmi le groupe consistant en peroxyde d'hydrogène, perborate, percarbonate, persulfate, perphosphate, peroxyde de calcium, peroxyde de magnésium et peroxyde d'urée.

6. Agent de blanchiment des dents selon la revendication 1, dans lequel (b) le composé chimique générant du peroxyde d'hydrogène dans une solution aqueuse est le peroxyde d'hydrogène.

7. Agent de blanchiment des dents selon la revendication 1, dans lequel la quantité de (b) le composé chimique générant du peroxyde d'hydrogène dans une solution aqueuse contenue dans l'agent est de 35 % en poids ou moins.

8. Agent de blanchiment des dents selon la revendication 1, dans lequel la quantité de (b) le composé chimique générant du peroxyde d'hydrogène dans une solution aqueuse contenue dans l'agent varie de 1 % en poids à 10 % en poids.

9. Agent de blanchiment des dents selon la revendication 1, dans lequel (c1) l'agent épaississant inorganique comprend au moins un type de minéral argileux inorganique choisi parmi le groupe consistant en saponite, montmorillonite, stevensite, hectorite, smectite, nacrite et sépiolite.

10. Agent de blanchiment des dents selon la revendication 1, dans lequel (c2) l'agent épaississant organique comprend au moins un type de matériau choisi parmi le groupe consistant en polyacrylate de sodium, méthylcellulose et carboxyméthylcellulose de sodium.

11. Procédé de blanchiment d'une dent par l'application de l'agent de blanchiment des dents selon l'une quelconque des revendications 1 à 10 sur la surface de celle-ci et par irradiation de la surface appliquée avec de la lumière.

12. Procédé selon la revendication 11, dans lequel la longueur d'onde de la lumière pour l'irradiation varie de 380 nm à 500 nm.
